# EUROPEAN PATENT SPECIFICATION

(11) **EP 1 937 177 B1**
(45) Date of publication and mention of the grant of the patent: **22.09.2010**
(21) Application number: 06818211.2
(22) Date of filing: 27.06.2006
(51) Int. Cl.: A61B 19/00

(54) **DEVICE AND METHOD FOR CONTROLLING A REMOTE APPLIANCE**
VORRICHTUNG UND VERFAHREN ZUR KONTROLLE EINER ENTFERNTEN VORRICHTUNG
DISPOSITIF ET PROCÉDÉ PERMETTANT DE COMMANDER UN APPAREIL DISTANT

(30) Priority: 20.09.2005 EP 05447210
(43) Date of publication of application: 02.07.2008
(73) Proprietor: Medsys S.A., B-5030 Gembloux (BE)
(72) Inventor: ANDRE, Jacques, B-1410 Waterloo (BE); FERRIERE, Laurent, B-1340 Ottignies (BE); POLET, Roland, B-6900 Marche-en-Fammene (BE)
(74) Representative: Michalík, Andrej
(86) International application number: PCT/EP2006/006217
(87) International publication number: WO 2007/038998

(56) References cited:
- EP-A- 1 253 509
- WO-A-97/49340
- WO-A-2005/039835
- DE-A1- 10 033 680
- US-A- 5 791 908
- US-A1- 2002 058 929
- US-B1- 6 377 011

## Description

### Technical field

The present specification relates to the field of minimally invasive surgery. In a first aspect the specification relates to a device for wireless control of a remote appliance e.g. an endoscope or the like in the cited field of application. In another aspect, the present specification also relates to a method for operating a remote appliance, by using a device according to the specification.

### Background

Minimally Invasive Surgery (MIS) is a new kind of surgery which gets more and more common nowadays. Another well-known expression is 'endoscopic surgery'. With this method, a surgical operation is performed by the help of a small endoscopic camera several long, thin, rigid instruments through natural body openings or small artificial incisions ('keyhole surgery'). The endoscope is basically a camera mounted on a long thin lens with a cable and a light source. The light source is mounted onto the lens and provides light to illuminate the field to be visualized. The cable mounted on the camera connects to a TV screen, which displays the camera's field of focus. The endoscope allows the surgeon to have an illuminated and magnified view of the operating field without having to make a large incision. With the assistance of the endoscope surgeons can utilize several small incisions to perform the same procedure they would otherwise perform using a single large incision. Particular techniques in endoscopic surgery include thorascopy, which is the visualization of the thoracic cavity or the chest, and laparoscopy, which is the visualization of the abdominal cavity.

In comparison to the usual, open surgery, there exist several advantages for the patient. Advantages of endoscopic surgery include for instance improved postoperative recovery, decreased pain, less strain of the organism, smaller injuries (aesthetic reasons), and faster return to activities and thus an economic gain (shorter illness time).

However, in an operation room, often many complex surgical instruments are available and have to be controlled and manipulated during endoscopic surgical procedures. A major problem associated with such endoscopic surgery is an inefficient coordination between the vision provided by the endoscope and the handling of the other remote surgical instruments, interfering in the endoscopic surgery. The quality of outcome of endoscopic surgery is highly dependent upon the view of the operative site throughout the procedure. Today, endoscopic surgeons have to rely on assistants and/or nursing personnel holding the endoscope to provide the optimum view of the procedure. However, even skilled assistants cannot always anticipate or interpret the surgeon's optimal view or next movements. Communication between surgeon and assistants may therefore often delay the procedure, lead to reduced efficacy, stress and less safe surgery.

WO 97/29690 discloses a robotic system for minimally invasive procedures, wherein robotic arms holding surgical instruments are remotely controlled by a surgeon using a joystick-operated device. However, in such system the surgeon's sense of touch and dexterity are highly reduced due to the use of long narrow endoscopic instruments, and precise motion of a tool at the tip of a surgical instrument may be lacking. In addition, with such systems a surgeon has no control over a visualising means such as endoscopic camera and still needs to rely on assistants and/or nursing personnel for holding the endoscope to provide the optimum view.

Further, DE 10 033 680 discloses a joystick-operated device for controlling appliances other than the surgical instrument held by the surgeon. Such device is attached directly to the surgeon's body, such as to his fingers or hand palm. However, the use of such devices limits the freedom of a surgeon to operate surgical instruments by the hand which holds the joystick-operated device.

EP 1 253 509 describes a joystick device which is incorporated into the handle of an instrument used for laparoscopic surgery, so that the top of the joystick is accessible to an operators finger for manipulation thereof. The joystick is able to translate spatial movements of a finger of a user into spatial movements of an object that is in wired or wireless connections with the joystick device. However, a major disadvantage of such system is that the joystick device is not adapted to be combined with various types of surgical instruments and cannot be readily, such as in the course of surgery, interchanged between different surgical instruments used by the operator. Moreover, the system disclosed in EP 1 253 509 only allows for a single, fixed position of the joystick relative to the handle of the surgical instrument in which it is incorporated. Therefore, the surgeon is not offered the option to easily adjust the location of the joystick relative to his hand, such as to make the joystick more accessible to the finger or fingers operating the joystick. Also, the system of EP 1 253 509 is especially suited for manipulating the joystick using the thumb, while the use of other fingers that may also be available or freed during surgery, may not be straightforward. EP 1 253 509 also teaches a joystick device worn by the surgeon on a digit of a finger, such as in the form of a ring. However, this arrangement requires that each time a surgeon wishes to switch hands during surgery, he must reposition the ring as well as any associated apparatus from one hand to the other, which may hamper the operation process.

In view of the above, it is therefore, clear that there remains a great need in the art for improved devices and methods for performing minimally invasive surgical procedures.

It is therefore an object of the present invention to provide an improved device for controlling the manipulation of surgical instruments and remote appliances.

It is in particular an object of the present invention to provide an improved device for controlling the manipulation of surgical instruments and one or more remote appliances, which are different from the hand-held surgical instrument.

It is another particular an object of the present invention to provide an improved device for controlling the manipulation of surgical instruments and one or more remote appliances, whereby the device may be adapted and used in combination with various types of surgical instruments.

### Summary

The present invention provides subject matter as set forth in any one and all of (i) to (xxiv) below:
(i) A kit of parts comprising:
   - a device (1) configured for producing signals suited for wireless control of a remote appliance, and comprising one or more input members (2) the manipulation of which causes the device (1) to produce the said signals, and
   - a clip (5) **characterised in that** said clip (5) is configured for releasable, clasping engagement with a hand-held surgical instrument and comprises a receiving surface (27) configured to receive and at least partly enclasp a portion of the hand-held surgical instrument, in particular a portion of the handle thereof or a portion distally adjacent to the handle, and further comprises, outwardly from the receiving surface (27), a housing member (19) and at least one supporting member (20) moveably engaged with the said housing member (19),
      wherein the device (1) and the supporting member (20) comprise mutually fitting connecting elements (10,9) for interconnection thereof.
(ii) A system adapted to perform surgery comprising:
   - a hand-held surgical instrument comprising distally an end effector and proximally a handle (3) for grasping by a user, and the manipulation of the handle actuates the end effector, and
   - the kit of parts as set forth in (i) above,
      wherein the receiving surface (27) of the clip (5) receives and at least partly enclasps a portion of the handle (3) or a portion of the surgical instrument distally adjacent to the handle (3), thereby releasably engaging the clip (5) with the hand-held surgical instrument, and wherein the device (1) and said supporting member (20) are interconnected through the mutually fitting connecting elements (10,9) provided respectively thereon.
(iii) A system or kit of parts as set forth in (i) or (ii) above, wherein the housing member (19) co-extends circumferentially with at least a part of the receiving surface (27) of the clip (5).
(iv) A system or kit of parts as set forth in (iii) above, wherein the supporting member (20) is moveable along at least a portion of the circumference of the respective housing member (19).
(v) A system or kit of parts as set forth in any one of (i) to (iv) above, wherein the supporting member (20) is moveable between two or more discrete positions on the housing member (19), or is continuously moveable along a given circumferential span of the housing member (19).
(vi) A system or kit of parts as set forth in any one of (i) to (v) above, wherein the supporting member (20) is slideably engaged with the housing member (19).
(vii) A system or kit of parts as set forth in any one of (i) to (vi) above, wherein the supporting member (20) defines a body generally protruding radially outwardly from the housing member (19), the said body defining a radially proximal end (34), a radially distal end (23) and an interposed stem portion (36) connecting the said ends.
(viii) A system or kit of parts as set forth in (vii) above, wherein the connecting element (9) is provided at or adjacent to the radially distal end (23) of the supporting member (20).
(ix) A system or kit of parts as set forth in (viii) above, wherein the connecting element (9) is moveable relative to the supporting element (20) on which it is provided.
(x) A system or kit of parts as set forth in any one of (i) to (ix) above, wherein the connecting elements (9,10) are configured for a moveable, preferably slideable, interconnection between the supporting member (20) and the device (1).
(xi) The system or kit of parts as set forth in any one of (i) to (x) above, wherein the device (1) comprises at least one pair, such as 2, 3 or more pairs, of input members (2) configured to produce identical signals, each member of said pair being positioned substantially symmetrically on the opposite lateral sides (4) of the device (1); preferably each pair is configured to produce signals different from any other pairs.
(xii) The system or kit of parts as set forth in any one of (i) to (x) above, wherein the device (1) comprises at least one pair of input members (2) configured to produce identical signals on the same lateral surface (4) of the device (1).
(xiii) The system or kit of parts as set forth in any one of (i) to (xii) above, wherein the said wireless control is effected by a transmitter-receiver system, preferably where the device comprises the transmitter and the appliance, or a second appliance configured to control the said appliance, comprises the receiver.
(xiv) The system or kit of parts as set forth in any one of (i) to (xiii) above, wherein the device is configured to control spatial movement of the remote appliance or a part thereof.
(xv) The system or kit of parts as set forth in any one of (i) to (xiv) above, wherein the remote appliance is chosen from the group comprising a positioning system for a surgical instrument, such as a positioning system for laparoscopic and/or endoscopic visualization systems, or a positioning system for load-carrying platforms.
(xvi) The system or kit of parts as set forth in any one of (i) to (xiv) above, wherein the device is configured to control a computer command effected by the remote appliance.
(xvii) The system or kit of parts as set forth in any one of (i) to (xvi) above, wherein manipulation of the one or more input members causes the device to produce signals suited to control the remote appliance.
(xviii) The system or kit of parts as set forth in any one of (i) to (xvii) above, wherein the manipulation comprises effecting spatial movement of and/or applying pressure on the one or more input members or parts thereof.
(xix) The system or kit of parts as set forth in any one of (i) to (xviii) above, wherein the one or more input members are positioned such that when the device is mounted to the hand-held surgical instrument, the input member can be manipulated by at least one finger of a hand holding the hand-held surgical instrument.
(xx) The system or kit of parts as set forth in any one of (i) to (xix) above, wherein the input member is chosen from the group comprising joystick, press button switch, touch pad or a rolling-ball.
(xxi) The system or kit of parts as set forth in (xx) above, wherein the joystick is configured to actuate spatial movement of the remote appliance or part thereof along 1 to 3 orthogonal axes.
(xxii) The system or kit of parts as set forth in (xx) above, wherein the device comprises at least one joystick configured to actuate spatial movement of the remote appliance or a part thereof along 2 orthogonal axes and at least one joystick configured to actuate spatial movement of the remote appliance or a part thereof along the third orthogonal axis.
(xxiii) The system or kit of parts as set forth in any one of (i) to (xxii) above, wherein the surgical instrument is a laparoscopic surgical instrument.
(xxiv) The system or kit of parts as set forth in any one of (i) to (xxiii) above, which is suitable for sterilization or is disposable.

The subject matter provided by the invention thus specifically belongs to the disclosure, description and teaching of the present specification.

The present specification is directed to an improved device for controlling the operation and the manipulation of surgical instruments and one or more remote appliances, in particular in the field of minimally invasive surgery. More in particular, the present specification describes improved devices adapted for simultaneously controlling the operation of surgical instruments and remote appliances, whereby said control may be effected by one and the same person, generally a surgeon.

In a first aspect, the specification therefore describes a device configured for wireless control of a remote appliance, the device comprising one or more input members. In particular, the device can produce signals that are suited for wireless control of the said remote appliance and the manipulation of the input members by the user causes the device to produce such signals.

The present device is in particular **characterized in that** it is configured for releasable mounting to a hand-held surgical instrument and that the remote appliance is distinct from the hand-held surgical instrument. The present surgeon-operated device for wireless control of a remote function has the following essential features.

The device is configured for releasable mounting to a hand-held surgical instrument. As used herein, releasable mounting refers to a situation in which the device can be coupled with a surgical instrument, by direct attachment therewith or indirectly joined via a suitably configured interposed member (e.g., a suitable adaptor means or a clip), the said interposed member connecting to both the device and the surgical instrument, wherein the said coupling can preferably be disengaged by an operating surgeon. Hence, the coupling may be susceptible to actions which are generally available to the surgeon, such as, e.g., pulling, pushing, cutting, tilting, rotating, etc. It is apparent that such release of the coupling may involve the release of a direct attachment between the device and the surgical instrument, or else where these are indirectly joined via an interposed member, the release of the connection between the device and the interposed member and/or the release of the connection between the interposed member and the surgical instrument. Hence, for example, a releasable mounting of the device to the surgical instrument may result from releasable coupling between the device and the interposed member and/or from a releasable coupling between the interposed member and the surgical instrument. For example, the user or an assistant may grasp the device and/or the interposed member and dismantle this from the surgical instrument.

In the present disclosure when the device is mounted or coupled to a surgical instrument as above, such mounting or coupling preferably creates a stable spatial association between the device and the surgical instrument, i.e., the continued maintenance of the spatial association does not in principle require any action of the operating surgeon, and the association is adequately stable not to substantially alter during normal use of the system in operation, unless of course the surgeon intentionally alters the position of the device relative to the surgical instrument.

It is appreciated that releasable mounting or coupling of the device to a surgical instrument enables the user or assistant to readily adapt the device among two or more different surgical instruments, such that the user keeps his control of the device-operated functions while switching between different surgical instruments. Moreover, in some embodiments the device may be releasably mounted on a given surgical instrument in various spatial associations relative to the instrument; in such instance, the possibility to readily release the coupling between the device and the instrument and subsequently re-couple the two in a different spatial relation may allow the operator to adjust the position of the device such as to make the device or its input members suitably accessible.

In preferred embodiments, releasable mounting between the device and the surgical instrument may be achieved via an adaptor means. In some embodiments, the adaptor means may be provided on the side configured to face the surgical instrument with clasping elements capable of claspingly at least partly embrace a portion of the surgical instrument.

Preferably, such clasping connection between the adaptor means and the surgical instrument may be releasable, such that the surgeon can position the adaptor means and thereby the device, e.g., to a different position on a same surgical instrument or to a different surgical instrument.

Accordingly, in embodiments, the adaptor means can claspingly engage with the surgical instrument in at least two different positions. Hence, by connecting the device to the so differentially positioned adaptor means, the device and so the input members thereof can be positioned differently relative to the instrument. The invention thus allows the user to control the spatial position of the device relative to the surgical instrument on which it is mounted, such as to improve the accessibility of the device and its input members by the surgeon.

For example, in an embodiment the clasping association between the adaptor means and a portion of the surgical instrument defines a cross-sectional area of the surgical element having a circumference, which is at least partly embraced by the clasping elements of the adaptor means. In an embodiment, the adaptor means may be capable of claspingly engaging with the said portion of the surgical instrument in two or more different angular positions relative to the said cross-sectional circumference. In such case, for example, if the device is provided at a certain circumferential position relative to the clasping elements of the adaptor means, the above engagement may allow to position the device at two or more different circumferential positions relative to the said cross-sectional circumference. This may help, e.g., to adjust the position of the device relative to the hand of a user.

Hence, the present specification also relates to a kit of parts comprising the said device and an adaptor means, wherein the adaptor means is configured to receive and at least partly embrace a portion of the surgical instrument. For example, in a preferred embodiment the adaptor means may comprise clasping elements, preferably one or more pairs of clasping elements, configured to claspingly engage with a portion of a surgical instrument, thereby releasably locking the adaptor means in a fixed position on the surgical instrument. The adaptor means and the device further comprise mutually fitting connecting elements for interconnection thereof; such connecting elements on the adaptor means can be typically provided opposite to, i.e., radially outwardly from the side which is configured to receive the surgical instrument.

A skilled person understands that the present teaching relates to the said kit of parts wherein the device and the adaptor means are not interconnected, as well as to such kit wherein the said parts are connected through the respective connecting elements. Moreover, the specification also foresees a system for performing surgery, wherein the said device with the adaptor means is releasably mounted on a surgical instrument.

The present device contains one or more input members which can be operated by a surgeon using the same hand in which the surgeon holds the hand-held surgical instrument. In addition, the remote function is executed by an appliance which is distinct from the hand-held surgical instrument. The present device thus provides the particular advantage that it enables a single person, generally a surgeon, to manipulate a surgical instrument by hand and at the same time to control a remote appliance by means of a wireless control system. The surgeon no longer has to rely on assistants and/or nursing personnel for holding an endoscope or other appliance to provide the optimum view of the procedure. Use of the present device in operation quarters may therefore put the surgeon back in control of vision and free an assistant or nurse for other tasks. Use of the present device also results in lower surgical risks, and eliminates unsolicited movements. In addition, use of the present device enables faster procedures and thus minimizes operating room costs to the patient. A further advantage is that by using the present device, the amount of time that a patient is under anesthesia, can be significantly reduced.

Another important advantage is that the present device can be mounted in a releasable way on a surgical instrument, and as such be used in combination with various types of surgical instruments.

In a further embodiment, the specification relates to a device, wherein the said wireless control is effected by a transmitter-receiver system, preferably wherein the device comprises the transmitter and the appliance, or a second appliance configured to control the said appliance, comprises the receiver. The present device is therefore particularly suitable to be operated in conjunction with many types of appliances, including but not limited to optical devices, such as endoscopic or laparoscopic cameras, positioning systems for patient-carrying platforms, computers, devices which may be operated by means of a vocal command etc... The present specification thus provides a device which is interchangeable and applicable in conjunction with many different types of appliances.

In another preferred embodiment, the specification provides a device wherein the one or more input members are positioned such that when the device is mounted to the hand-held surgical instrument, the input member can be manipulated by at least one finger of a hand holding the hand-held surgical instrument. When holding the hand-held surgical instrument, a surgeon always has at least one finger which remains free, for instance the index finger, and which can be used for manipulating the input member(s) on the device. A surgeon has sufficient degree of freedom to more dexterously and precisely manipulate the device.

In another further embodiment, the specification provides a device comprising at least one pair, such as 2, 3 or more pairs, of input members configured to produce identical signals, each member of said pair being positioned substantially symmetrically on the opposite lateral sides of the device; preferably each pair is configured to produce signals different from any other pairs. By symmetrically arranging the input members on both sides of the device, the present device is advantageously suitable for both left- and right-handed surgeons. In addition, by configuring each pair of input members to produce signals different from any other pairs, the device advantageously provides a possibility to remotely control more than one appliance.

In another embodiment, input members configured to produce an identical signal may also be included on the same surface of the device. Such configuration may allow the surgeon to choose to use the input member which is generally more easily accessible to his fingers in a particular situation. For example, he may choose to use the input member which is closer in the direction of a movement of a finger, e.g., when extending or retracting the finger.

In a yet further embodiment, input members configured to produce different signals can be suitably distinguished from each other by their shape. This allows the surgeon to tell apart such input members tactilely without having to look at the device, which improves his concentration on the operating task. This embodiment may be particularly advantageous if such input members are positioned on the same surface of the device. In another embodiment, the specification is directed to a device configured for wireless control of a remote appliance, wherein the input member(s) are adapted to actuate spatial movement of a remote appliance or part thereof along 1 to 3 orthogonal axes. This advantageously allows a surgeon to control movements of appliances along all orthogonal axes x, y, and z.

As noted, the releasable mounting of a device is effected by means of a suitable adaptor means. Preferably said adaptor means is specifically configured for releasable or permanent interconnection with the hand-held surgical instrument, preferably adjacent to the proximal end and/or handle of the instrument. Depending on the configuration of this adaptor means, the present device may be easily and rapidly mounted in a releasable way on various kinds of hand-held surgical instruments. The present specification thus describes a simple device having tool changing capabilities so that various tools or surgical instruments may be easily and readily replaced to enable faster procedures and minimize operation costs.

The specification to a method for controlling the manipulation of a hand-held surgical instrument and a remote appliance, whereby said remote appliance is distinct from the surgical instrument, said method comprising the steps of:
a) mounting a device configured for wireless control of a remote appliance and comprising one or more input members to said hand-held surgical instrument,
b) holding the surgical instrument in a hand for manipulation thereof, and
c) controlling said remote appliance by manipulating the input member(s) of said device by at least one finger of the hand holding the hand-held surgical instrument.

Preferably the specification describes a method wherein the operation of said hand-held surgical instrument and said remote appliance is controlled simultaneously by a single person.

Further advantageous aspects which may further improve the working and flexibility of the system.

Accordingly, in a further aspect, the invention provides a system adapted to perform surgery comprising:
- a hand-held surgical instrument comprising distally an end effector and proximally a handle for grasping by a user, and the manipulation of the handle actuates the end effector,
- a device configured for producing signals suited for wireless control of a remote appliance, and comprising one or more input members the manipulation of which causes the device to produce the said signals, and
- a clip comprising a receiving surface which receives and at least partly enclasps a portion of the handle or a portion of the surgical instrument distally adjacent to the handle, thereby releasably engaging the clip with the hand-held surgical instrument; the clip further comprising, outwardly from the receiving surface, a housing member and at least one supporting member moveably engaged with the said housing member,
   wherein the device and the clip are interconnected through mutually fitting connecting elements provided respectively on the device and on the said supporting member.

The above aspect provides for further advantages related to the way in which the device as taught herein is mounted to the hand-held surgical instrument. In particular, due to the moveable engagement between the supporting member, which is connected with and bears the device, and the housing member provided on the clip, the user may change the position of the device relative to the clip. Hence, while the clip may be suitably engaged in one or more alternative fixed positions on the hand-held surgical instrument, the user can readily move the supporting member and therewith the device relative to the clip and thus relative to the surgical instrument, esp. without disengaging the contact between the clip and the surgical instrument. This way, the distance between the hand of the user and the one or more input members provided on the device can be easily adjusted to differently sized hands of users before and/or during use, or can allow the user to reposition the device when switching hands or changing the manner of grasping the handle of the surgical instrument.

In the above aspect, the housing and supporting members are provided outwardly from the receiving surface of the clip. In this context, the term "outwardly" denotes a direction which is substantially opposite to the direction from the receiving surface of the clip towards the portion of the surgical instrument embraced thereby. Relative to the cross-sectional area at least partly embraced by the receiving surface, the term "outwardly" thus has the meaning radially outwardly, i.e., radially away from the receiving surface.

A skilled person will understand that the present specification also relates to particular parts of the above system, especially to the device and the clip as such. The invention further relates to a kit of parts comprising the device and the clip in both an interconnected and standalone configuration. Accordingly, in an aspect, the invention relates to a kit of parts comprising:
- a device configured for producing signals suited for wireless control of a remote appliance, and comprising one or more input members the manipulation of which causes the device to produce the said signals, and
- a clip configured for releasable, clasping engagement with a hand-held surgical instrument and comprising a receiving surface configured to receive and at least partly enclasp a portion of the hand-held surgical instrument, in particular a portion of the handle thereof or a portion distally adjacent to the handle, and further comprising, outwardly from the receiving surface, a housing member and at least one supporting member moveably engaged with the said housing member,
   wherein the device and the clip comprise mutually fitting connecting elements, provided respectively on the device and on the said supporting member, for interconnection thereof.

A skilled person can appreciate that the invention relates to the above kit of parts, wherein the parts are provided separately or in an interconnected configuration.

With the insight to better show the characteristics and advantages of the invention, some preferred embodiments and examples are described hereafter referring to the enclosed figures.

### Description of the figures

**Figure 1** represents an embodiment of a device as taught herein configured for wireless control of a remote appliance as mounted in a releasable way to the handle of a hand-held surgical instrument.
**Figure 2** and **3** illustrate embodiments of a device as taught herein configured for wireless control of a remote appliance.
**Figure 4** represents the internal structure of an embodiment of a device as taught herein configured for wireless control of a remote appliance.
**Figure 5** shows an embodiment of the kit of parts (comprising device and clip) releasably mounted on a hand-held endoscopic surgical instrument.
**Figure 6** shows the slideable engagement between the device and the supporting member of a clip comprised in the kit of parts.
**Figure 7** shows embodiments of the device as taught herein having input members only on one lateral side, or duplicated on both lateral sides.
**Figure 8** shows embodiments of the clip of the kit of parts; (illustrated without a closing member) comprising one or two circumferentially extending housing members, and respective supporting members.
**Figure 9** shows front and cross-sectional views of one embodiment of the clip of the kit of parts comprising a closing member.
**Figure 10** illustrates exemplary embodiments of the supporting member used in a preferred embodiment of the invention.

### Detailed description of the invention

The present specification describes an improved device for performing minimally invasive surgery. In particular, the present specification relates to a surgeon-operated device for wireless control of a remote function. The present device is adapted to effect wireless control of a remote appliance. The device comprises one or more input members and is configured for releasable mounting to a hand-held surgical instrument or the handle thereof. It is in particularly noted that the remote appliance, controlled by the present device, is distinct from the hand-held surgical instrument. In accordance with the present invention, the remote function is thus executed by an appliance, instrument and/or device distinct from the hand-held surgical instrument. Preferably the remote function is carried out by an appliance which is not physically connected with the surgical instrument.

The present device is very compact and allows a completely wireless operation, thus obviating the necessity of being attached to a device which is to be controlled. The term *"hand-held instrument',* as used herein includes that the instrument is directly held by a surgeon close to its proximal end and that contacts a patient's body at its distal end. The hand-held surgical instrument may be any type of surgical instrument which may be suitable for performing minimally invasive surgery. Suitable examples of hand-held surgical instruments may include but are not limited to instruments adapted to cut or incise, to retract, to grasp/hold/occlude, to dilate or probe, to cannulate or drain, to aspirate/inject/infuse, to suture or ligate. In a preferred example, the hand-held surgical instrument is a laparoscopic surgical instrument.

A hand-held surgical instrument, and a hand-held endoscopic or laparoscopic surgical instrument in particular, may comprise an end effector, typically configured for performing any of the above surgical actions, and proximally a handle for grasping by a user, wherein the manipulation of the handle actuates the end effector. Endoscopic and laparoscopic instruments may typically further comprise an elongated tube or shaft which encloses a relay means, e.g., a suitable wire, for relaying the movements of the handle to the end effector. Hence, the end effector-shaft-handle line defines the "longitudinal" axis of such an instrument. Endoscopic and laparoscopic instruments may have any of the types of handles commonly employed in the art for that purpose, such as, e.g., scissor-like handles, pistol-trigger handles, etc. Such handles may typically comprise two pivotally-connected arms or members, wherein pivoting of the arms relative to one another actuates the end-effector through a suitable relay mechanism. As used herein, the plane in which the said arms of the handle are situated is referred to as "vertical" plane of the handle or of the surgical instrument.

The *"remote appliance"* may be chosen from the group comprising but not limited to a positioning system for a surgical instrument, such as a positioning system for laparoscopic and/or endoscopic visualization systems, or a positioning system for load-carrying platforms, such as patient-carrying platforms, computers, etc... In a preferred embodiment, the device as taught herein is configured to control spatial movement of the remote appliance or a part thereof. In another embodiment, the present device as taught herein is configured to control a computer command effected by the remote appliance.

The *"input member"* of the device as taught herein may be chosen from the group comprising joystick, press button switch, touch pad or a rolling-ball. The corresponding manipulation of the input member by a user actuates the device to generate signals for wirelessly controlling the remote appliance.

Accordingly, the present specification also discloses a device, wherein manipulation of the one or more input members causes the device to produce signals suited to control the remote appliance. Preferably said, manipulation comprises effecting spatial movement of and/or applying pressure on the one or more input members or parts thereof. The one or more input members may be operated by a surgeon using the same hand in which he holds the hand-held surgical instrument. For this, in an embodiment, the input member(s), e.g. a joystick(s), are positioned on the device such that when the device is mounted to the hand-held surgical instrument, the input member(s) can be manipulated by at least one finger of a hand holding the hand-held surgical instrument, for instance the index finger. The user's hand is in no way hampered during operation; a simple lifting of the finger is enough to interrupt the ongoing movements without any delay and thus without errors.

The input member(s) on the device preferably are joysticks which translate spatial movements of the surgeon's finger into spatial movements or other actions of the remote appliances.

In one embodiment, the specification describes a device, wherein the input member, e.g. a joystick, is configured to actuate spatial movement of the remote appliance or part thereof along 1 to 3 orthogonal axes. Alternatively, a device as taught herein may comprise at least one input member, e.g. a joystick, configured to actuate spatial movement of the remote appliance or a part thereof along 2 orthogonal axes and at least one (other) joystick configured to actuate spatial movement of the remote appliance or a part thereof along the third orthogonal axis.

The device may comprise 1, 2, 3, or more pairs of input members configured to produce signals. In an example, the device comprises at least two input members on at least one lateral side of the device. In another example, the device comprises at least two input members on both laterals sides thereof. In a preferred example, the device may advantageously contain 2 joysticks whereby at least 6 degrees of freedom are achieved. This allows the surgeon to control movements of appliances along all orthogonal axes x, y, and z or for instance in the directions left, right, up, down, into patient, away from patient. Preferably, joysticks controlling these functions are placed on the same side of the device and can be easily operated by the surgeon using the same finger. Further, it shall be noted that the joysticks are preferably arranged symmetrically on both sides of the device, such that the device is suitable for both left- and right-handed surgeons.

In one embodiment, the one or more pairs of input members are configured to produce identical signals. In another embodiment, each pair may be configured to produce signals different from any of the other pairs. For instance, the joystick is able to transmit 2 to 6 distinguishable commands, and/or 2 to 6 degrees of freedom. Preferably, transmission begins when the joystick is pressed or moved in a certain direction and stops when the joystick is released.

In one embodiment, the input member(s) on the device preferably are joysticks which translate spatial movements of the surgeon's finger into spatial movements of the remote appliances. In a preferred embodiment, a device as taught herein is operated by one finger. More particularly, movement in six directions may be commanded by one finger.

The specification is however also related to variants of the above described device, wherein a number of movements are commanded by other means. In another embodiment, the input member(s) on the device preferably are joysticks which translate spatial movements of the surgeon's finger into other actions, e.g. no spatial movements of the remote appliances. These other actions or other means preferably consist of a vocal command. Known techniques for translating vocal commands into command signals may be used for this. In all devices which have this capability however, an on/off switch has to be present, commanding the activation or the interruption of the vocal command itself. This introduces a security element in the device's operation: for example by pressing a button, the vocal command of a number of movements is enabled, and by releasing said button, this vocal command is disabled. As an example of such a device, an embodiment of the present device may consist of a device as defined herein, wherein one or more input members act as controllers of one or more vocal commands. In an example, this vocal command may be able to control the up-down movement, for example, while the left-right and in-out movement are still being commanded by a finger movement, e.g. by means of manipulating other joystick(s). Activating the input member allows 'up-down' to be controlled vocally. Releasing the input member blocks the vocal command.

In a preferred embodiment, releasable mounting of the device to a surgical instrument is done by means of a suitable adaptor means. Such adaptor means may be an integral part of and attached to the device. In a preferred embodiment, the adaptors means as taught herein is configured for permanent interconnection with the hand-held surgical instrument. In such embodiment, the specification describes device configured for wireless control of a remote appliance, comprising one or more input members and further comprising an adaptor means which is permanently connected to the said device.

Alternatively, the adaptor means may be separately provided, and may be adapted to be connectable to the device in a releasable way. In a preferred embodiment, the adaptor means as taught herein is therefore configured for releasable interconnection with the hand-held surgical instrument. In another preferred embodiment, the device as taught herein is configured for releasable, interconnection with the adaptor means.

Preferably said adaptor means is specifically configured for releasable or permanent interconnection with the hand-held surgical instrument, preferably adjacent to the proximal end and/or handle of the instrument.

The present device may be connected to various types of adaptors means for mounting on a surgical instrument. The configuration of such adaptor means may advantageously be chosen in order to fit on a particular type of surgical instrument. The adaptors means may be adapted to be mounted either on the proximal end of a surgical instrument or on the handle of the instrument, such that for every type of surgical instrument a suitable adaptor means is provided. In function of the configuration of the adaptor means, the present device may thus be easily mounted on various types of surgical instruments.

In a preferred embodiment the adaptor means is provided on its side facing the surgical instrument with one or more clasping elements, for clasping a surgical instrument or handle thereof. In addition, the adaptor means is provided, on its side facing the device with connecting elements for connection to the device.

In a preferred embodiment, the adaptor means comprise one or more, and preferably at least two pairs of clasping elements, e.g. legs. Preferably said pairs of clasping elements are spatially separated. By means of these clasping elements an adaptor means according to the present invention may be locked in a fixed position on a surgical instrument. These clasping elements enable clasping of the adaptor means on a surgical instrument, e.g. the handle thereof, by frictional engagement. The clasping elements thereby provide sufficient grip of the adaptor means, and the device connected thereto, on the instrument, but at the same time remain sufficiently loose to allow easy removal of the adaptor means, and the thereto connected device, from the surgical instrument. The present adaptor means preferably consists of one or more materials, e.g. synthetic material such as PP, PE or the like, which are suitable for allowing the clasping elements, e.g. legs, to able to undergo slight deformation when adapting the adaptor means to a surgical instrument, but which nevertheless still provide sufficient tightening of immobilization. In a preferred embodiment, the clasping elements of the adaptors means are further provided with a notch or the like to improve fitting of the adaptors means on the surgical instrument or the handle thereof, and thus to improve fixation of the adaptor means on the surgical instrument or the handle thereof.

In another embodiment, the adaptor means is provided with connecting elements which correspond to and fit with connecting elements provided on the device. In a preferred embodiment, the connecting elements of the adaptor means comprise a guide rail or groove system, which correspond to connecting elements on the device such as e.g. longitudinal notches, guide rail or groove system, provided on the underside of the device and enabling a longitudinal adjustment of the position of the device on the adaptor means. The present adaptor means and device are particularly suitable for being slidably engaged into one another, in particular by means of the corresponding connecting elements provided on the adaptor means and the device.

The adaptor means is generally placed on top of the hand-held instrument and pressed towards the instrument for gripping therein. By a rotating movement, with respect to the vertical longitudinal plane of the surgical instrument, the adaptor means can be locked on the instrument. Such locked position may result in a tilting of the device attached to the adaptor means circumferentially laterally by for instance from -60° to +60°, and preferably from -45° to +45° with respect to the vertical longitudinal plane of the surgical instrument. Preferably, stable, locked positions of the device are symmetrically located with respect to the vertical longitudinal plane of the surgical instrument, such that an operator, when changing hands during the manipulation procedure, can easily adapt the device accordingly to operate by either hand holding the surgical instrument.

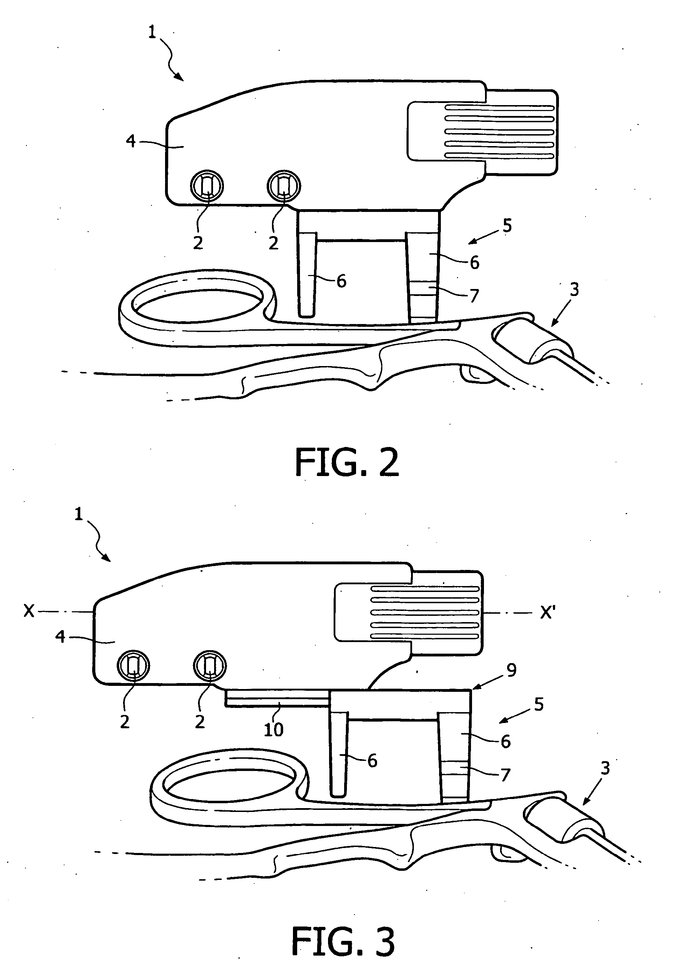

Furthermore, the present device is perfectly adaptable to the hand structure and dimensions of a user. For this, in another embodiment, the device which can be mounted at two or more different distances from the proximal end and/or handle of the hand-held surgical instrument. For adapting the above mentioned positions, the present device may be provided with connecting elements such as e.g. notches, which fit into connecting elements of the adaptor means, e.g. a guide rail or groove system. Such connecting elements allow the device to be slidably engaged in longitudinal direction along the groove or rail of the adaptor means. The device may slide forwards or backwards on the adaptor means to accommodate larger or smaller surgeon's hands. For instance, depending on the position of the device on the adaptor means, the device may be provided in backwards position, as illustrated on **figure 2****,** in which position it is particularly suitable for being manipulated by a person having shorter fingers. The device may also be provided in forward position, as shown on **figure 3****,** in which a larger hand. It shall be clear however, that other position of the device than those illustrated in the figures can as well be obtained. For instance, the device can be stopped at different positions when being interconnected with the adaptor means, said different positions providing different distances of at least one input member to the hand of an operator in which he holds the device. In an example, the device can be configured at a position such that the most distal input member can be reached by a substantially stretched finger of an operator, preferably its index finger. Advantageously, the position of the device on the hand-held surgical instrument can be easily adapted even during manipulation of the device and the instrument by an operator.

It is essential that surgical instruments are sterilized before or after use. Advantageously, the present specification is also directed to a device, as disclosed herein, which is suitable for sterilization, e.g. sterilization by steam. As such, the present specification thus provides a device which may advantageously be sterilised together with surgical instruments, and which does not need separate handling. In addition, repeated washing and sterilization of surgical instruments tends to quickly destroy many materials. However, the present specification describes a device which can be used up to more than 20 times, preferably even up to at least 30 times, before having to replace it. Alternatively, the present device and or adaptor means / clip may be provided to the user in sterilised (e.g., plasma sterilised) single package units, and may be disposed after having been employed in surgery.

In another embodiment, the present specification further provides a kit comprising a device as taught herein and one or more, preferably different types of adaptor means, such as clip(s) according to the invention, which may be adapted for releasable connection to said device.

In a further development, a system is provided adapted to perform surgery comprising:
- a hand-held surgical instrument comprising distally an end effector and proximally a handle for grasping by a user, and the manipulation of the handle actuates the end effector,
- a device configured for producing signals suited for wireless control of a remote appliance, and comprising one or more input members the manipulation of which causes the device to produce the said signals, and
- a clip comprising a receiving surface which receives and at least partly enclasps a portion of the handle or a portion of the surgical instrument distally adjacent to the handle, thereby releasably engaging the clip with the hand-held surgical instrument; the clip further comprising, outwardly from the receiving surface, a housing member and at least one supporting member moveably engaged with the said housing member,
   wherein the device and the clip are interconnected through mutually fitting connecting elements provided respectively on the device and on the said supporting member.

A skilled person will appreciate that this configuration enables a further freedom of mobility of the device relative to the handle of the surgical instrument, but that the here above described preferred properties of the device, surgical instrument or structures of the clip, such as, for example, connecting elements on the device and on the clip, also apply to this aspect of the invention.

The present specification relates to particular parts of the above system, especially to the device and the clip as such, as well as to a kit of parts comprising the device and the clip in both an interconnected and standalone configuration. It shall also be understood that where this specification explains preferred features of the invention with reference to the above system, such features also apply herein to the individual parts of the system, especially to the device and the clip as such, and a kit of parts comprising the device and the clip, as taught by the present specification.

Accordingly, in an embodiment, the invention relates to a kit of parts comprising:
- a device configured for producing signals suited for wireless control of a remote appliance, and comprising one or more input members the manipulation of which causes the device to produce the said signals, and
- a clip configured for releasable, clasping engagement with a hand-held surgical instrument and comprising a receiving surface configured to receive and at least partly enclasp a portion of the hand-held surgical instrument, in particular a portion of the handle thereof or a portion distally adjacent to the handle, and further comprising, outwardly from the receiving surface, a housing member and at least one supporting member moveably engaged with the said housing member,
   wherein the device and the clip comprise mutually fitting connecting elements, provided respectively on the device and on the said supporting member, for interconnection thereof.

The term "clip" as used herein generally refers to a member configured to at least partly embrace, grip, clasp, or hook an object, such as herein, a portion of a surgical instrument, thereby frictionally engaging or locking therewith.

As used herein, a clip may enclasp only a part of the circumference of the respective object. For example, a clip may comprise clasping arms or legs capable of exerting sufficient pressure radially inwardly on the enclasped surgical instrument as to frictionally engage the clip therewith. For example, a clip may comprise one or more pairs of such clasping arms. Alternatively or in addition, a clip may be provided with a closing means configured to join the circumferential endings of the clasping elements such as to provide a closed loop arrangement embracing the whole circumference of the enclasped object. Preferably, such closing means can be suitably tightened in order to hold the clasping arms tightly around the enclasped object. In an embodiment, the clip may comprise clasping arms or a clasping member configured to at least partially enclasp a portion of the surgical instrument, wherein the clasping arms / member by itself may but need not exert a sufficient inward pressure on the enclasped object; rather, the tight grip of the clip on the object may be achieved by tightening of the circumferential ends of the clasping arms / member with a suitable closing means. An exemplary closing means may be, e.g., a suitable flexible strap, band, lash, belt, tether, or strip, such as a single use unidirectional strap.

In an embodiment, the clip may be configured to claspingly embrace a cross sectional area between about 10mm2 and 800mm2, preferably between 20mm2 and 500mm2. In another embodiment, this cross-sectional area may have a circumference of between 10mm and 100mm.

The clip defines a receiving surface specifically adapted to receive and at least partly enclasp a portion of the surgical instrument. Thus, when the clip is secured on a surgical instrument, at least a part of the receiving surface contacts and frictionally engages the corresponding circumferential surface of the surgical instrument.

In a particular embodiment, the housing member co-extends circumferentially with at least a part of the receiving surface of the clip. In other words, the clip can be provided with a housing member extending between two different circumferential positions defined on the receiving surface. The housing member may but need not extend substantially parallel to the said circumferentially co-extending portion of the receiving surface.

As noted, the receiving surface of the clip at least partly encircles or is configured to encircle the portion of the surgical instrument which it receives or is configured to receive. Accordingly, a housing member which co-extends circumferentially along at least a part of the said receiving surface will also co-extend with at least a part of the circumference of the portion of the surgical instrument which is embraced by the clip. Such housing member may thus advantageously allow the movement of the supporting member and the device along such circumferential trajectory.

Accordingly, in a further embodiment the supporting member is moveable along at least a portion of the circumference of the respective housing member with which it is moveably engaged. As explained, this allows the supporting member and the device connected thereto to be moved about the circumference of the housing member, thereby positioning the device at various circumferential locations relative to the portion of the surgical instrument embraced by the clip. Hereby, the device and its one or more input members may be moved closer or further away to a user hand gripping the handle of the surgical instrument. Moreover, with an adequate circumferential span of such movement, the device may be suitably positioned at circumferential locations which can be reached by the fingers of, respectively, the right user hand or the left user hand; this may allow for switching of hands during the use of the system, as well as for easy adaptation of the system for use by both right- and left-handed users.

In the present invention, the supporting member may be moveable relative to the respective housing member in numerous ways. By means of example and not limitation, the supporting member may be moveable between two or more discrete positions on the housing member, and it may remain stably interlocked with the housing member in such positions (i.e., in the said positions the supporting member remains interlocked with the respective housing member during normal use without needing to be held in such positions by the user, but can be readily moved from such interlocked position to another one by the user). The supporting member may be moveable between such stably interlocking positions while being continuously in contact with the housing member, or else may need to be disassociated from the housing member in order to move it between the different positions. Alternatively, the supporting member may be continuously moveable along a given circumferential span of the housing member and may remain stably interlocked with the housing member at any position within such span.

Accordingly, in an exemplary, preferred embodiment, the supporting member may be slideably engaged with the housing member. Suitable arrangements for slideable engagement of elements are known in the art and can be employed in the present invention, such as various groove and guide rails systems, etc. Again, such slideable engagement may allow for a continuum of or, alternatively, for a discrete number of positions in which the supporting member is stably interlocked with the housing member.

In a further embodiment the supporting member defines a body generally protruding radially outwardly from the housing member (i.e., radially away from the housing member, as opposed to radially towards the receiving surface and the surgical instrument embraced thereby). This may advantageously increase the distance between the housing member and the point of attachment of the device on the supporting member, thereby avoiding potential steric hindrance between the device and other parts of the system (e.g., surgical instrument, clip, housing element) during the movement of the device. By means of an illustrative and non-limiting example as shown in the figures, the clip may be connected to a section of the surgical instrument which has a smaller cross-section or diameter than another, relatively adjacent portion of the instrument (e.g., here the rotary wheel). In that situation, inclusion in the system of a supporting member which projects radially away from the housing member may advantageously achieve that the device will be positioned radially further away from the body of the clip and will thus not interfere in its movement with nearby sections of the surgical instrument having a greater cross-section or diameter (e.g., the rotary wheel). In addition, placing of the device radially further outwardly from the clip may also help to suitably position the device vis-à-vis the hand of the user.

Generally, the body of the supporting member may comprise a radially proximal end, i.e., an end radially closer to the supporting member and the body of the clip 5, and a radially distal end, i.e., an end radially farther away from the supporting element and the body of the clip, and an interposed stem portion connecting the said ends. The radially proximal end of the supporting member may be suitably configured to mediate the moveable engagement between the supporting member and the housing member of the clip. The connecting element can be preferably provided at or adjacent to the radially distal end of the supporting member.

A skilled person can appreciate that the interposed stem portion of the supporting member may be readily provided in different shapes, sizes, lengths, etc., whereby substantially like designs of a clip and/or of the device may be adapted to various shapes and types of surgical instruments or handles thereof, such as to achieve an optimal placing of the input members of the device relative to the hand of a user.

In an embodiment, the connecting element may be provided in a fixed position relative to the supporting element, such that the device will be specifically aligned relative to the clip and the surgical instrument when interconnected with the said connecting element. The connecting element may be suitably so positioned as to achieve optimal accessibility of the input members of the device by the user.

In another embodiment, the connecting element may be configured to allow for movement, such as, e.g., a tilting or rotary movement, relative to the supporting element on which it is provided. Such configuration may allow for further freedom in placing the device relative to the surgical instrument, such as to maximize the accessibility of the input members by the user. By means of an example and not limitation, the radially distal end of the supporting member may be connected to the stem portion of the same by means of a suitable joint, pivot or hinge element allowing the movement of the said end relative to the stem.

In a further embodiment, the connecting elements may be configured to provide for only one way of interconnection between the device and the supporting member.

Alternatively, in another embodiment, the connecting elements may allow for moveable, and preferably slideable, interconnection between the said structures. For example, the device may be provided with connecting elements such as e.g. notches, which fit into connecting elements of the clip, e.g. a guide rail or groove system. Such connecting elements allow the device to be slideably engaged along the groove or rail of the supporting member of the clip. Once again, such slideable engagement may provide a further control of positioning the device and its input members relative to the user hand.

In an exemplary, preferred configuration, the connecting elements can permit the device to slide at a given angle (e.g., perpendicularly or substantially perpendicularly) relative to the cross-sectional area embraced by the receiving surface of the clip. In many system configurations, this can result in sliding of the device in a direction having a component parallel to the proximal-to-distal direction of the surgical instrument. This sliding may further accommodate for the use of the system by users with different hand and finger sizes.

In an embodiment, the device may comprise one input member.

In a further embodiment, the device may comprise two or more input members. As detailed, the inclusion of two or more input members may allow for controlling different outcomes. Alternatively or in addition, two or more pairs of input members wherein each member of such a pair controls the same action. The respective members of the pair may be placed on the same surface of the device, or substantially symmetrically on opposite surfaces of the device, as explained elsewhere in this specification in connection to the device.

As further explained, input members for different functions may be distinguished by different shapes.

Generally, the wireless controller device may define a bottom or base surface radially proximal to the clip and typically comprising the connecting element for interconnection with the connecting element of the supporting member, and an opposite top surface, which in some embodiments may also comprise a connecting element, to allow for various ways of attaching the device to the supporting member.

The device further defines opposite lateral surfaces. Typically, when the device is connected with the supporting element, the lateral surfaces may be substantially perpendicular to the cross-sectional area enclosed by the clip and may extend at different circumferential positions relative to the receiving surface of the clip. Preferably, the input members may be provided at these lateral surfaces, which might be best accessible by the surgeon operating the system.

The invention is here after illustrated with reference to the enclosed figures, which intend to improve the understanding of the various features of the invention, but are not to be considered limiting in any way.

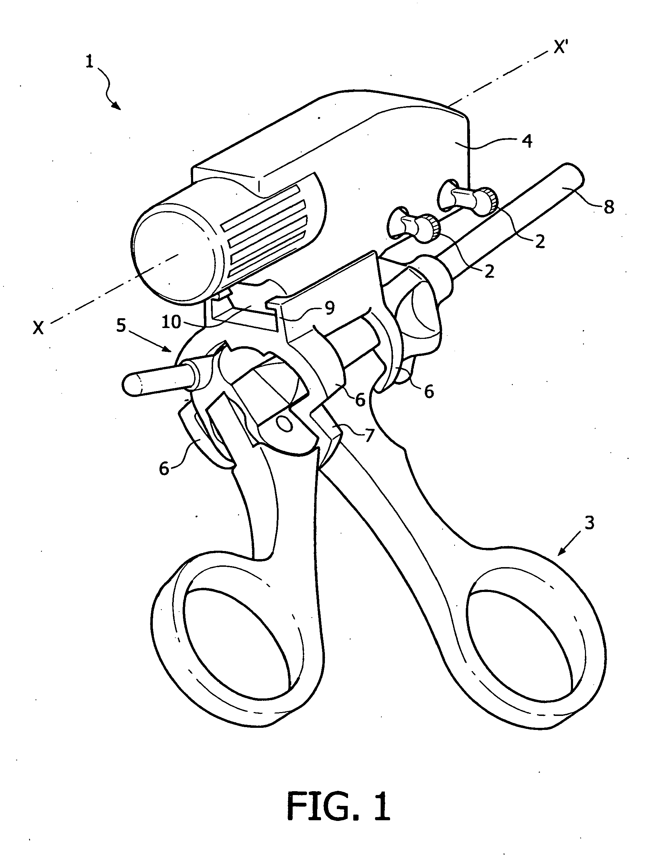

In reference to **figure 1****,** a device 1 as taught herein is illustrated. The represented device comprises two pairs of joystick the lateral sides 4 of the device 1. In figure 1, only one lateral side of the device is illustrated showing two joysticks 2. It shall be clear that the opposite lateral side of the device (not shown) in an equal manner comprises two joysticks which are arranged symmetrically with respect to the axis X-X'. The device 1 is clasped in a releasable way on the handle of a hand-held surgical instrument 3 by means of an adaptor means 5. The handle of a hand-held surgical instrument may be further provided with connecting means 8 for attaching or connecting a surgical instrument, e.g. a laparoscopic instrument. The adaptor means 5 consists of two pairs of clasping elements 6 which are spatially separated and suitable for grasping the handle of the instrument 3. The clasping elements of the adaptors means 6 are further provided with a notch 7 for better fitting of the elements on the handle and thus to improve the mounting of the adaptor means 5 on the handle 3. The adaptor means 5 are further provided with connecting elements 9, in particular a groove system 9, which corresponds with and fits to connecting elements 10 provided on the lower side of the device 1.

Referring now to **figures 2** and **3****,** a joystick-operated device 1 configured for mounting on a hand-held surgical instrument 3 is illustrated. The represented device 1 comprises two pairs of joystick like input members 2, which are provided symmetrically with respect to the axis X-X' on both lateral sides 4 of the device 1. Again, in figures 2 and 3, the input members 2 on only one lateral side 4 of the device are represented. The corresponding input members of the pairs are provided symmetrically on the opposite lateral side of the device (not shown). An adaptor means 5 for mounting of the device 1 on a surgical instrument 3 is attached to the device 1. The adaptor means 5 consists of two pairs of clasping elements which are spatially separated and further provided with a notch 7 for further fitting of the elements on the handle 3. Figure 3 further illustrates how the device 1 is provided with connecting elements 10, e.g. a notch or a guide rail or groove system 10, for connection with the connecting elements 9 of adaptor means 5, e.g. a guide rail or groove system 9. This type of connection allows the device to slide along the adaptor means and to take in different positions. For instance, in figure 2 the device 1 is provided in backwards position on the adaptor means 5, while in figure 3 the device 1 is provided in forward position. Such type of connection also provides for easy adjustment and removal of the device from the adaptor means.

In a preferred example, the present device is a coded wireless control unit, positioned on the handle of a surgical instrument having two joysticks symmetrically provided on both sides of the device for ergonomic access with the finger of a user and able to manage 6 movements of a remote appliance such as a laparoscope, i.e. in/out, left/right and up/down, which can be activated with left and right hand.

In a preferred embodiment, the present device has a weight which is comprised between 50 and 150 gram, and preferably between 60 and 100 gram, and for instance of 70 gram. In another preferred embodiment, the present device has dimensions of between 4.25 cm X 1.5 cm X 1 cm and 12.75 cm X 4.5 cm X 3 cm, and for instance of 8.5 cm X 3cm X 2 cm.

In another preferred embodiment, the wireless control of a device according to the invention is effected by a transmitter-receiver system. Preferably, the device comprises the transmitter and the appliance instrument and/or device, or a second appliance instrument and/or device configured to control the first appliance instrument and/or device, comprises the receiver. The appliance, instrument and/or device performing the remote function, which is operated by means of the present device, are preferably able to emit a sensory signal when performing the remote function.

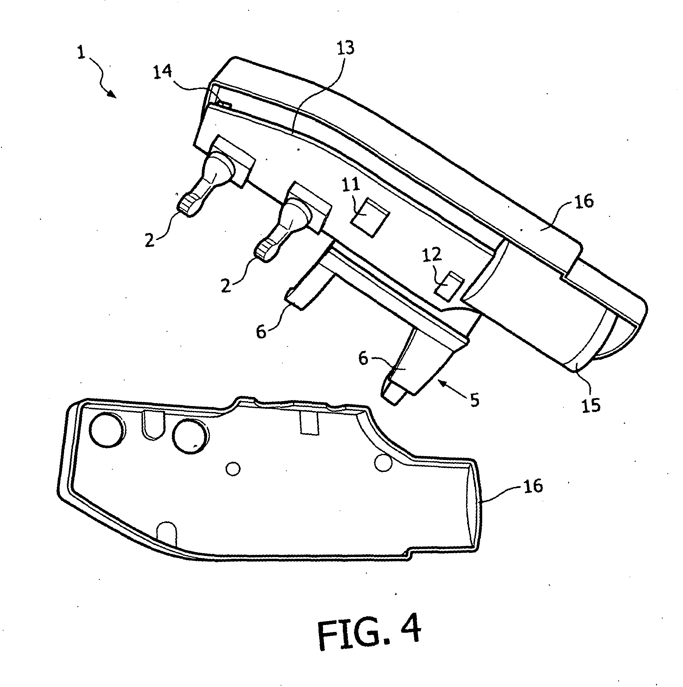

In a preferred embodiment, the present device comprises a transmitter, a controller, a battery, an internal antenna, software means, and a power supply. Preferably said elements are provided within a housing. In another preferred embodiment, the battery of the device may be a replaceable or built-in battery. In another preferred embodiment, the internal antenna is tied to an open printed circuit board (PCB) antenna that runs around the outside edge of a PCB provided in the device. In an example, **figure 4** represents the internal structure of an embodiment of a device 1 according to the invention configured for wireless control of a remote appliance. The device may be mounted in a releasable way to a hand-held surgical instrument by means of adaptor means 5, and operated by means of input members 2. As represented on figure 4, a controller 11, a transmitter 12, a PCB 13 on which an internal antenna 14 is provided, and a battery 15 are provided within housing 16 of the device 1.

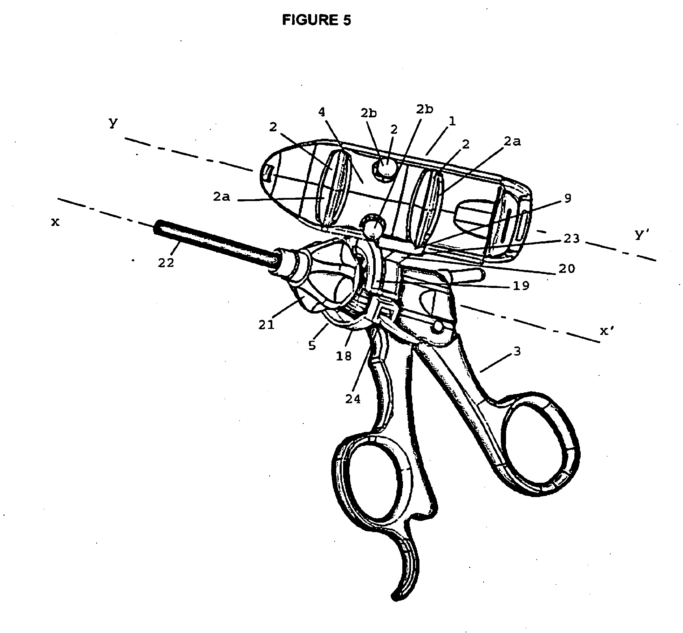

Figures 5 through 10 show further embodiments of the device, clip, operating system and kit op parts as taught herein. A skilled person will understand that the above described characteristics, e.g., relating to the device, the connecting elements between the adaptor means and the device, the way of attachment of the adaptor means to the surgical instrument, etc. may also be applied to the embodiments explained in the following.

**Figure 5** illustrates a system adapted to perform endoscopic surgery comprising a joystick operated device 1 as taught herein, mounted on the handle 3 of an endoscopic surgical instrument by means of a clip 5. The handle is substantially a scissor-like handle comprising pivoting arms configured for grasping by a surgeon. The handle 3 further comprises a rotary wheel 21 which serves to control the rotary movement of the effector end (not shown) provided on the distal end of the shaft 22. Herein, the clip 5 is secured circumferentially around a portion of the handle 3 distal to the arms of the handle 3 and proximal to the rotary wheel 21. The cross-sectional area encircled by the receiving surface (i.e., the radially proximal surface) of the clip 5 is substantially perpendicular to the longitudinal axis X-X' of the surgical instrument. The positioning of the clip 5 does not hinder a surgeon in accessing and controlling the rotary wheel 21.

The clip 5 encircles the circumference of the portion of the handle 3 on which it is mounted and is tightened around the said circumference using a suitable closing or tightening means 18, such as herein illustrated a flexible strap, e.g., a single use, unidirectional strap. The closing means 18 may take the form of any substantially flexible member capable of effecting a closed loop between the circumferential end portions of other members of a clip 5. The closing means 18 comprises a closing mechanism 24 which allows the closing means 18 with the rest of the clip 5 to form the said closed loop around the handle 3. The closing mechanism 24 may be any known in the art (e.g., a pin-and-whole arrangement, a clamp, etc.) and may preferably allow for releasable engagement with different positions on the closing means 18. The clip 5 further comprises a housing member 19 and engaged moveably therein a supporting member 20. The radially distal end 23 of the supporting member comprises connecting element 9 for fittingly interconnecting the supporting member 20 with a corresponding connecting element provided on the joystick device 1.

As illustrated herein, the longitudinal axis Y-Y' of the joystick device 1 may be substantially parallel to the longitudinal axis X-X' of the surgical instrument. In examples, the two axes may be slanted towards each other at an angle, e.g., between 0° and 60°, preferably between 0° and 30°, such as between 0° and 15°. Moreover, the device 1 displays lateral surfaces 4 substantially parallel to the longitudinal axis Y-Y' of the device 1. The said lateral surface 4 of the device 1 may as shown herein be substantially perpendicular to the cross-sectional area encircled by the receiving surface of the clip 5.

The said lateral surface 4 comprises several input members 2. In the shown configuration, a surgeon holding the handle 3 in his left hand, e.g., passing his middle finger through the opening in the more distal arm of the handle 3 and his thumb through the more proximal arm of the handle, may control the input members 2 on the device 1 using his index finger. A symmetrical engagement of the device 1 across the longitudinal axis X-X' with a therein provided supporting member 20 would make the joysticks accessible to a right hand user.

It is a property of the invention that the lateral surface 4 of the device 1 comprising the input members 2 is moveable circumferentially with respect to the cross-sectional area encircled by the clip 5. In the illustrated embodiment, this movement would alter the spatial orientation and/or the distance of the lateral surface 4 of the device 1 and the input members 2 relative to the handle 3 of the surgical instrument. This would effect the distance between the hand of a surgeon and the input members 2, thereby making the system adjustable to make the input members comfortably accessible to the operator.

Herein, the device 1 comprises differently shaped joysticks 2 on the displayed lateral surface 4, i.e., elongated joysticks 2a and ball-ending joysticks 2b, wherein joysticks of different shape may preferably control different signals generated by the device 1, such that the surgeon can differentiate the joysticks and the actions they control by touch. Also herein, the device comprises two elongated joysticks 2a at different positions along the longitudinal axis Y-Y' of the device 1 on the lateral surface 4 of the device 1. The said elongate joysticks 2a may be set to control the same action, such that the surgeon is allowed to choose the joystick 2a which may be more easy to access and manipulate in a particular situation, to control the said action.

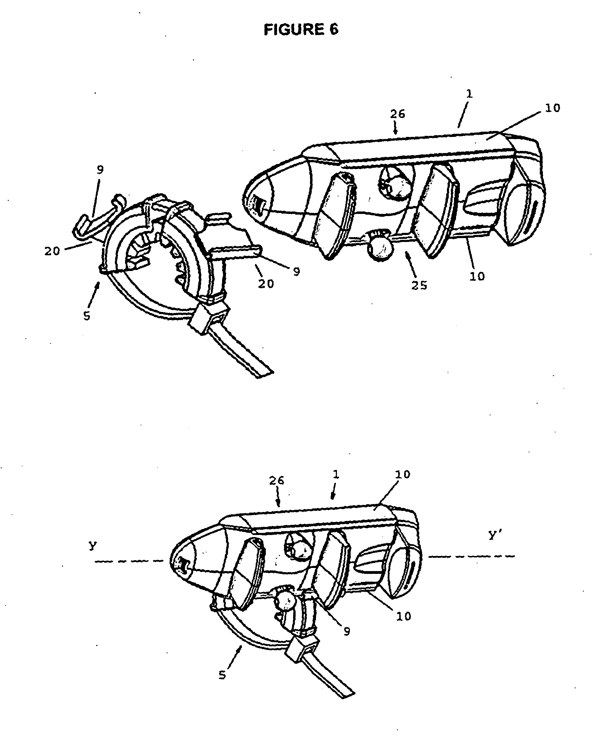

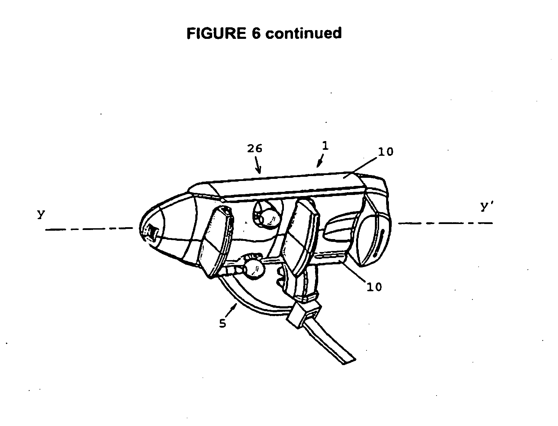

Moving to **figure 6**, this illustrates slideable engagement between the connecting element 9 provided on the supporting member 20 and having essentially a configuration of guide grooves, and a complementarily fitting connecting element 10 provided on the base surface 25 of the device 1 having essentially a configuration of guide rails. A skilled person understands that various other means of slideable arrangement between the above members may also be used in the invention. The said slideable arrangement may define a continuum of different positions in the sliding direction and/or may define several positions of relatively more stable engagement between the connecting elements 9,10 along the sliding direction.

As shown, the sliding direction of the device 1 may be substantially perpendicular to the cross-sectional area defined by the clip 5, or in examples, be at an angle to the cross-sectional area defined by the clip 5 of between 30° and 90° and preferably between 60° and 90°. Applying this slideable movement to the embodiment shown in Figure 5, this may position the device 1 and its input members 2 at different distances from the handle 3 along the longitudinal axis of the surgical instrument X-X'. This further helps to adjust the system to different hand sizes of operators by allowing to regulate the distance from the hand along the axis X-X'.

Note that a similar connecting element 10 is also provided on the top surface 26 of the device, allowing a symmetrical engagement across the longitudinal axis X-X' with the other connecting element 9.

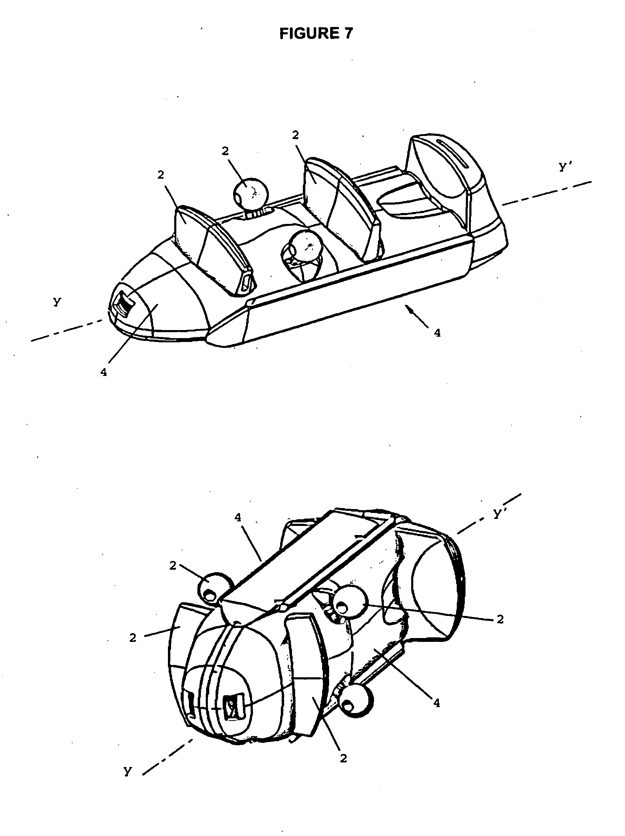

**Figure 7** illustrates possible embodiments of the device 1. The device on top includes input members 2 only on one of its lateral surfaces 4. In use, such as in the system illustrated in Figure 5, such device may need to be inverted symmetrically across its longitudinal axis Y-Y' to allow for use with both right and left hand.

The bottom device includes pairs of input members 2 substantially symmetrically positioned on its opposite lateral surfaces 4, and for controlling the same action. In the system of Figure 5, such device 1 may be moved circumferentially relative to the cross-sectional area enclosed by the clip 5, e.g., between positions closer to, respectively, the left and right lateral sides of the handle 3, such that the input members 2 on the opposite lateral surfaces 4 of device 1 would become accessible to, respectively, the left and right hands of a surgeon.

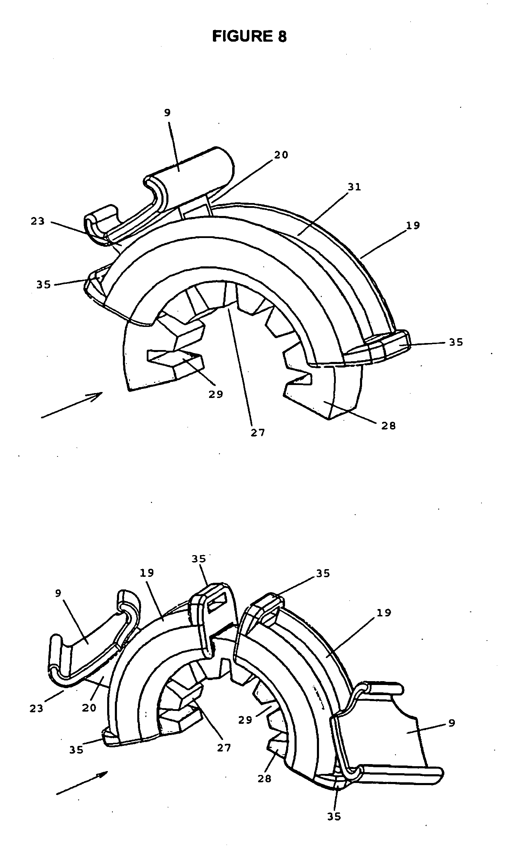

Turning to **figure 8****,** this illustrates two of possible arrangements of the clip 5, herein shown without the closing member 18. In the top embodiment, the clip 5 comprises a receiving surface 27, herein provided by a substantially arcuate inner member 28. A substantially arcuate shape of the member 28 may be capable of engaging differently shaped surgical handles, but other geometries of the member 28 are be possible, e.g., to account for particular handle shapes. The member 28 may be preferably made of relatively flexible material, e.g., silicone or rubber foam, in order to obtain a close fit between the receiving surface of a clip 5 and the surgical handle 3. The circumferentially spaced corrugations 29 on the radially inner side of the member 28 may facilitate radially inward or outward bending of the member 28, to accommodate different handles. The member 28 may have a circumferential length, e.g., between 0.5cm and 10cm, such as between 2cm and 6cm, to engage with a sufficient portion of a handle 3.

Radially outwardly and attached to the member 28 is provided the housing member 19, in which the supporting member 20 is moveably arranged circumferentially. The herein illustrated attachment between the member 28 and the housing member 19 is by a fitting relationship, in which the radially outer portion of the member 28 fits into a complementary profile, herein a groove, provided on the radially inner surface of the housing member 19. Other means of such attachment, e.g., by adhesives or mechanical means, are also contemplated. The housing member 19 may have a circumferential length, e.g., between 0.5cm and 10cm, such as between 2cm and 6cm, to embrace a sufficient portion of a handle 3. The circumferential length of the housing member 19 may be preferably the same or smaller than that of the inner member 28.

In the illustrated embodiment, the supporting member 20 comprises a radially proximal end 34 which slideably fits into a circumferentially extending groove 31 provided on the radially outer side of the housing member 19. The groove 31 in the housing member 19 may comprise circumferentially positioned stopping members 35 to limit the circumferential movement of the supporting member 19 to within the groove 31.The supporting member 20 comprises at its radially distal end 23 a connecting element 9 configured to slideably engage the connecting element 10 of the device 1. The housing member 19 may be advantageously made of substantially rigid material, e.g., substantially rigid plastic such as PE, PP, etc., such that the groove 31 provided therein would not be deformed and loose functionality when the clip 5 is positioned on the surgical instrument 3. Similarly, the supporting member 20 may be made of substantially rigid material, e.g., substantially rigid plastic such as PE, PP, etc., e.g., to provide for firm holding of the device 1.

In the bottom of figure 8, an embodiment of the clip 5 is illustrated which comprises two housing members 19 arranged circumferentially adjacently relative to each other and connected there between by a relatively flexible portion of the clip 5, e.g., by the inner member 28. This arrangement allows for the clip 5 to be easily bent radially inwardly or outwardly, even when the housing members 19 are themselves relatively rigid, thereby receiving various diameters of surgical instruments 3. Preferably, each housing member comprises a supporting member 20 moveably engaged along its circumference. Applying this embodiment to the system in Figure 5, such clip 5 may be symmetrically seated on the top side of a surgical handle 3, such that one of the housing members 19 extends towards one lateral side of the handle 3, while the other housing member 19 extends towards the opposite lateral side of the handle 3. Hence, this provides for a circumferentially moveable supporting member 20 on each lateral side of the handle 3, thus allowing the system to be used with both the right and left hands.

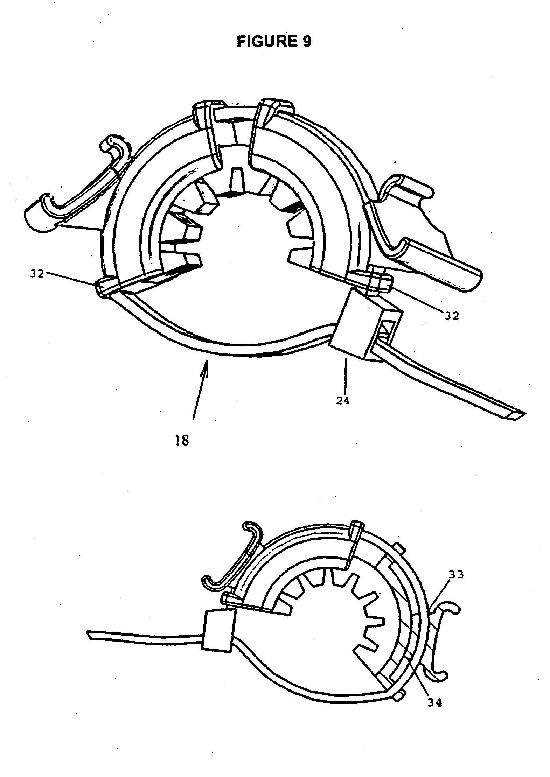

**Figure 9** shows the clip 5 as illustrated in Figure 8 bottom, but with the closing means 18 forming a closed loop by virtue of the closing mechanism 24. As clearly follows from the illustration, the closing means 18 fulfils additional functions in this embodiment. In particular, besides providing a closed loop between the circumferential extremes 32 of the housing members 19 of the clip 5, the closing means 18, which in this case is a suitable, flexible strap, extends along the outer circumference of the housing members 19 and fittingly within the groove 31 provided in the housing members 19. Moreover, the supporting members 20 are adapted to receive the closing member 18 through a circumferentially extending opening 33 in their radially proximal ends 34. Hereby, the tightening of the clip 5 using the closing means 18 around a surgical instrument also pushes the supporting members 20 radially inwardly against housing member 19, esp. against the base of the groove 31, thus increasing the frictional engagement between these members and allowing for stable maintenance of the supporting members 20 when moved between different circumferential positions along the said groove 31.

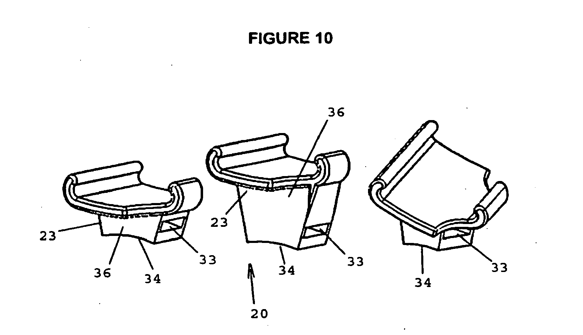

Finally, **figure 10** illustrates some of the different shapes and sizes of the supporting members 20, wherein the connecting element 9 provided at the radially distal end 23 of the supporting members 20 is visible, as is the through-going opening 33 in the radially proximal ends 34. The illustrated supporting members 20 may differ, e.g., in their radial length preferably in the length of the stem portion 36 between their radially opposite ends, and/or in the positioning of the connecting element 9 relative to the stem portion, etc.

As a skilled person will appreciate, the above explained preferred features will apply not only to the present system comprising the device 1, clip 5 and surgical instrument, but also to the device 1 and clip 5 as such and a kit of parts comprising the device 1 and clip 5 in separately or in an interconnected configuration.

In a further aspect, the specification relates to the use of any system or kit of parts as explained above, or of the device 1 and/or clip 5 as explained above in surgery.

In another aspect, the specification relates to a method for controlling the manipulation of a hand-held surgical instrument and a remote appliance, whereby said remote appliance is distinct from the surgical instrument comprising the steps of:
a) mounting the present kit op parts comprising the device 1 and the clip 5 to a hand-held surgical instrument,
b) holding the surgical instrument in a hand for manipulation thereof, and
c) controlling said remote appliance by manipulating the input member(s) 2 of said device 1 by at least one finger of the hand holding the hand-held surgical instrument

In an example of operation, when a user tilts a joystick, the embedded electronics and software of the device send a radio frequency data packet message, which is a function of the position of the joystick.

In another preferred operation mode, the present device is further adapted to control transmission of signals, preferably by radio transmission, dependent on the manipulation effected on the input member(s). For instance, when an input member is pressed continuously for more than e.g. 3 seconds, the device stops signal transmission and does not transmit any further signals until all input members are released and at least one input member is pressed again. Such operational command prevents unwanted permanent transmission of data when the joystick(s) are accidentally pressed or blocked in an active state.

The present device is in particular further adapted to transmit an observable or detectable warning when approaching the end of its life time. In an example, the present device is adapted to internally store the length of time it has been used, i.e. actual signal transmission. When it reaches a certain time, e.g. 100 minutes of use, it may start warning a user that it approaches end of life. When the end of life has been reached, e.g. 110 minutes, the present device is configured to stop transmitting completely.

**Table 1** represents some technical characteristics of a preferred embodiment of a device according to as taught herein.

**Table 1**

| | |
|---|---|
| Maximum supply voltage | 6 VDC |
| Supply current with joysticks released | 0mA |
| Total supply current when active | 10mA |
| Working frequency | 433.92MHz |
| Autonomy in normal use (NEAREND) | 6000seconds |
| Extra autonomy (ENDOFLIFE) | 600seconds |
| Operating temperature | 0 to +55°C |
| Storage temperature | 0 to +70°C |
| Recommended storage temperature | 0 to +40°C |
| Expected storage lifetime at recommended temperature | 2 years |

The present specification thus provides a device which permits to provide simultaneous control of one or more remote appliances and a surgical instrument.

## Claims

1. A kit of parts comprising:
- a device (1) configured for producing signals suited for wireless control of a remote appliance, and comprising one or more input members (2) the manipulation of which causes the device (1) to produce the said signals, and
- a clip (5) **characterised in that** said clip (5) is configured for releasable, clasping engagement with a hand-held surgical instrument and comprises a receiving surface (27) configured to receive and at least partly enclasp a portion of the hand-held surgical instrument, in particular a portion of the handle thereof or a portion distally adjacent to the handle, and further comprises, outwardly from the receiving surface (27), a housing member (19) and at least one supporting member (20) moveably engaged with the said housing member (19),
wherein the device (1) and the supporting member (20) comprise mutually fitting connecting elements (10,9) for interconnection thereof.

2. A system adapted to perform surgery comprising:
- a hand-held surgical instrument comprising distally an end effector and proximally a handle (3) for grasping by a user, and the manipulation of the handle actuates the end effector, and
- the kit of parts according to claim 1,
wherein the receiving surface (27) of the clip (5) receives and at least partly enclasps a portion of the handle (3) or a portion of the surgical instrument distally adjacent to the handle (3), thereby releasably engaging the clip (5) with the hand-held surgical instrument, and wherein the device (1) and said supporting member (20) are interconnected through the mutually fitting connecting elements (10,9) provided respectively thereon.

3. A system or kit of parts according to claims 1 or 2, wherein the housing member (19) co-extends circumferentially with at least a part of the receiving surface (27) of the clip (5).

4. A system or kit of parts according to claim 3, wherein the supporting member (20) is moveable along at least a portion of the circumference of the respective housing member (19).

5. A system or kit of parts according to any of claims 1 to 4, wherein the supporting member (20) is moveable between two or more discrete positions on the housing member (19), or is continuously moveable along a given circumferential span of the housing member (19).

6. A system or kit of parts according to any of claims 1 to 5, wherein the supporting member (20) is slideably engaged with the housing member (19).

7. A system or kit of parts according to any of claims 1 to 6, wherein the supporting member (20) defines a body generally protruding radially outwardly from the housing member (19), the said body defining a radially proximal end (34), a radially distal end (23) and an interposed stem portion (36) connecting the said ends.

8. A system or kit of parts according to claim 7, wherein the connecting element (9) is provided at or adjacent to the radially distal end (23) of the supporting member (20).

9. A system or kit of parts according to claim 8, wherein the connecting element (9) is moveable relative to the supporting element (20) on which it is provided.

10. A system or kit of parts according to any of claims 1 to 9, wherein the connecting elements (9,10) are configured for a moveable, preferably slideable, interconnection between the supporting member (20) and the device (1).

11. The system or kit of parts according to any of claims 1 to 10, wherein the device (1) comprises at least one pair, such as 2, 3 or more pairs, of input members (2) configured to produce identical signals, each member of said pair being positioned substantially symmetrically on the opposite lateral sides (4) of the device (1); preferably each pair is configured to produce signals different from any other pairs.

12. The system or kit of parts according to any of claims 1 to 10, wherein the device (1) comprises at least one pair of input members (2) configured to produce identical signals on the same lateral surface (4) of the device (1).

13. The system or kit of parts according to any of claims 1 to 12, wherein the said wireless control is effected by a transmitter-receiver system, preferably where the device comprises the transmitter and the appliance, or a second appliance configured to control the said appliance, comprises the receiver.

14. The system or kit of parts according to any of claims 1 to 13, wherein the device is configured to control spatial movement of the remote appliance or a part thereof.

15. The system or kit of parts according to any of claims 1 to 14, wherein the remote appliance is chosen from the group comprising a positioning system for a surgical instrument, such as a positioning system for laparoscopic and/or endoscopic visualization systems, or a positioning system for load-carrying platforms.

16. The system or kit of parts according to any of claims 1 to 14, wherein the device is configured to control a computer command effected by the remote appliance.

17. The system or kit of parts according to any of claims 1 to 16, wherein manipulation of the one or more input members causes the device to produce signals suited to control the remote appliance.

18. The system or kit of parts according to any of claims 1 to 17, wherein the manipulation comprises effecting spatial movement of and/or applying pressure on the one or more input members or parts thereof.

19. The system or kit of parts according to any of claims 1 to 18, wherein the one or more input members are positioned such that when the device is mounted to the hand-held surgical instrument, the input member can be manipulated by at least one finger of a hand holding the hand-held surgical instrument.

20. The system or kit of parts according to any of claims 1 to 19, wherein the input member is chosen from the group comprising joystick, press button switch, touch pad or a rolling-ball.

21. The system or kit of parts according to claim 20, wherein the joystick is configured to actuate spatial movement of the remote appliance or part thereof along 1 to 3 orthogonal axes.

22. The system or kit of parts according to claim 20, wherein the device comprises at least one joystick configured to actuate spatial movement of the remote appliance or a part thereof along 2 orthogonal axes and at least one joystick configured to actuate spatial movement of the remote appliance or a part thereof along the third orthogonal axis.

23. The system or kit of parts according to any of claims 1 to 22, wherein the surgical instrument is a laparoscopic surgical instrument.

24. The system or kit of parts according to any of claims 1 to 23, which is suitable for sterilization or is disposable.

## Patentansprüche

1. Kit-of-Parts, umfassend:
- eine Vorrichtung (1), die zur Erzeugung von Signalen konfiguriert ist, welche zum drahtlosen Steuern eines ferngesteuerten Gerätes geeignet sind, und die eine oder mehrere Eingabeelemente (2) enthält, deren Bedienung die Erzeugung der Signale durch die Vorrichtung (1) verursacht, und
- eine Klammer (5), **dadurch gekennzeichnet, dass** die Klammer (5) so konfiguriert ist, dass sie mit einer freigebbaren Umklammerung mit einem chirurgischen Handinstrument im Eingriff steht und eine Aufnahmefläche (27) aufweist, die zum Aufnehmen und mindestens teilweisen Umfassen von einem Teilbereich des chirurgischen Handinstruments konfiguriert ist, insbesondere einem Teilbereich von dem Griffstück davon oder einem Teilbereich, der distal an das Griffstück angrenzt, und ferner, von der Aufnahmefläche (27) nach außen gerichtet, ein Gehäuseelement (19) und mindestens ein Trägerelement (20) aufweist, das mit dem Gehäuseelement (19) beweglich im Eingriff steht, wobei die Vorrichtung (1) und das Trägerelement (20) gegenseitig passende Verbindungselemente (10, 9) zur Verbindung davon aufweisen.

2. System, das zur Durchführung von Operationen geeignet ist, umfassend:
- ein chirurgisches Handinstrument, das distal eine Greifvorrichtung und proximal ein Griffstück (3) zum Ergreifen durch einen Benutzer aufweist, und die Bedienung von dem Griffstück die Greifvorrichtung betätigt, und
- das Kit-of-Parts nach Anspruch 1,
wobei die Aufnahmefläche (27) von der Klammer (5) einen Teilbereich von dem Griffstück (3) oder einen Teilbereich, der distal an das Griffstück (3) anschließt, von dem chirurgischen Instrument aufnimmt und mindestens teilweise umklammert, wodurch die Klammer (5) lösbar mit dem chirurgischen Handinstrument im Eingriff steht, und wobei die Vorrichtung (1) und das Trägerelement (20) durch die gegenseitig passenden Verbindungselemente (10, 9), die entsprechend darauf bereitgestellt sind, miteinander verbunden sind.

3. System oder Kit-of-Parts nach Anspruch 1 oder 2, wobei sich das Gehäuseelement (19) zusammen mit mindestens einem Abschnitt der Aufnahmefläche (27) der Klammer (5) in Umfangsrichtung erstreckt.

4. System oder Kit-of-Parts nach Anspruch 3, wobei das Trägerelement (20) entlang von mindestens einem Teilbereich von dem Umfang des entsprechenden Gehäuseelementes (19) beweglich ist.

5. System oder Kit-of-Parts nach einem der Ansprüche 1 bis 4, wobei das Trägerelement (20) zwischen zwei oder mehr diskreten Positionen von dem Gehäuseelement (19) beweglich oder entlang einer vorgegebenen Umfangserstreckungsrichtung von dem Gehäuseelement (19) kontinuierlich bewegbar ist.

6. System oder Kit-of-Parts nach einem der Ansprüche 1 bis 5, wobei das Trägerelement (20) mit dem Gehäuseelement (19) verschiebbar im Eingriff steht.

7. System oder Kit-of-Parts nach einem der Ansprüche 1 bis 6, wobei das Trägerelement (20) einen Aufbau definiert, der im Allgemeinen von dem Gehäuseelement (19) radial nach außen gerichtet hervorsteht, wobei der Aufbau durch ein radiales proximales Ende (34), ein radial distales Ende (23) und einen eingeschobenen Schaftabschnitt (36), der die Enden verbindet, **gekennzeichnet** ist.

8. System oder Kit-of-Parts nach Anspruch 7, wobei das Verbindungselement (9) an oder anschließend an das radial distale Ende (23) von dem Trägerelement (20) vorgesehen ist.

9. System oder Kit-of-Parts nach Anspruch 8, wobei das Verbindungselement (9) relativ zu dem Trägerelement (20), an dem es bereitgestellt ist, beweglich ist.

10. System oder Kit-of-Parts nach einem der Ansprüche 1 bis 9, wobei die Verbindungselemente (9, 10) für eine bewegliche, vorzugsweise verschiebbare, Verbindung zwischen dem Trägerelement (20) und der Vorrichtung (1) konfiguriert sind.

11. System oder Kit-of-Parts nach einem der Ansprüche 1 bis 10, wobei die Vorrichtung (1) mindestens ein Paar, wie etwa 2, 3 oder mehr Paare, an Eingabeelementen (2) aufweist, die für die Erzeugung identischer Signale konfiguriert sind, wobei jedes Element von dem Paar im wesentlichen symmetrisch an den gegenüberliegenden Längsseiten (4) der Vorrichtung (1) positioniert ist; jedes Paar ist vorzugsweise so konfiguriert, dass es Signale erzeugt, die zu irgendeinem der anderen Paare unterschiedlich sind.

12. System oder Kit-of-Parts nach einem der Ansprüche 1 bis 10, wobei die Vorrichtung (1) mindestens ein Paar an Eingabeelementen (2) aufweist, das für die Erzeugung identischer Signale an der gleichen Seitenfläche (4) von der Vorrichtung (1) konfiguriert ist.

13. System oder Kit-of-Parts nach einem der Ansprüche 1 bis 12, wobei die drahtlose Steuerung durch ein Sender-Empfänger-System ausgeführt wird, vorzugsweise weist dabei die Vorrichtung den Sender auf und das Gerät, oder ein zweites Gerät, das für die Steuerung des Gerätes konfiguriert ist, weist den Empfänger auf.

14. System oder Kit-of-Parts nach einem der Ansprüche 1 bis 13, wobei die Vorrichtung zur Steuerung der räumlichen Bewegung von dem ferngesteuerten Gerät oder einem Teil davon konfiguriert ist.

15. System oder Kit-of-Parts nach einem der Ansprüche 1 bis 14, wobei das ferngesteuerte Gerät ausgewählt ist aus der Gruppe, die ein Positionierungssystem für ein chirurgisches Gerät umfasst, wie beispielsweise ein Positionierungssystem für laparoskopische und/oder endoskopische Visualisierungssysteme oder ein Positionierungssystem für Transportplattformen.

16. System oder Kit-of-Parts nach einem der Ansprüche 1 bis 14, wobei die Vorrichtung zur Steuerung eines Computerbefehls, der durch das ferngesteuerte Gerät ausgeführt wird, konfiguriert ist.

17. System oder Kit-of-Parts nach einem der Ansprüche 1 bis 16, wobei die Bedienung von einem oder mehreren Eingabeelementen die Vorrichtung dazu veranlasst, Signale zur Steuerung des ferngesteuerten Gerätes zu produzieren.

18. System oder Kit-of-Parts nach einem der Ansprüche 1 bis 17, wobei die Bedienung die Ausführung räumlicher Bewegungen von und/oder das Anwenden von Druck auf eines oder mehrere der Eingabeelemente oder Teilbereiche davon umfasst.

19. System oder Kit-of-Parts nach einem der Ansprüche 1 bis 18, wobei das eine oder die mehreren Eingabeelemente derart positioniert sind, dass, wenn die Vorrichtung auf dem chirurgischen Handinstrument montiert ist, das Eingabeelement mindestens mit einem Finger von einer Hand, welche das chirurgische Handinstrument hält, bedient werden kann.

20. System oder Kit-of-Parts nach einem der Ansprüche 1 bis 19, wobei das Eingabeelement ausgewählt ist aus der Gruppe, die Joystick, Drucktastenschalter, integriertem Berührungsfeld oder einer Rollkugel umfasst.

21. System oder Kit-of-Parts nach Anspruch 20, wobei der Joystick zur Ausführung räumlicher Bewegungen von dem ferngesteuerten Gerät oder Teilbereichen davon entlang von 1 bis 3 orthogonalen Achsen konfiguriert ist.

22. System oder Kit-of-Parts nach Anspruch 20, wobei die Vorrichtung mindestens einen Joystick, der zur Ausführung räumlicher Bewegungen von dem ferngesteuerten Gerät oder eines Teilbereichs davon entlang von 2 orthogonalen Achsen konfiguriert ist und mindestens einen Joystick aufweist, der zur Ausführung räumlicher Bewegungen von dem ferngesteuerten Gerät oder eines Teilbereichs davon entlang der dritten orthogonalen Achse konfiguriert ist.

23. System oder Kit-of-Parts nach einem der Ansprüche 1 bis 22, wobei das chirurgische Instrument ein laparoskopisches chirurgisches Instrument ist.

24. System oder Kit-of-Parts nach einem der Ansprüche 1 bis 23, das zur Sterilisation geeignet oder ein Einmalartikel ist.

## Revendications

1. Kit de pièces, qui comprend :
- un dispositif (1) configuré pour produire des signaux adaptés à la commande sans fil d'un appareil distant, et qui comprend un ou plusieurs éléments d'entrée (2) dont la manipulation entraîne la production par le dispositif (1) desdits signaux, et
- une pince (5) **caractérisée en ce que** ladite pince (5) est configurée pour se mettre en prise par pincement, de manière libérable, avec un instrument chirurgical manuel et comprend une surface de réception (27) configurée pour recevoir et au moins partiellement serrer une partie de l'instrument chirurgical manuel, en particulier une partie de sa poignée ou une partie distalement adjacente à la poignée, et comprend en outre à l'extérieur de la surface de réception (27), un élément de logement (19) et au moins un élément de support (20) en prise mobile avec ledit élément de logement (19),
le dispositif (1) et l'élément de support (20) comprenant des éléments de connexion s'emboîtant mutuellement (10, 9) pour s'interconnecter.

2. Système adapté pour réaliser une opération chirurgicale, qui comprend :
- un instrument chirurgical manuel qui comprend distalement un effecteur terminal et proximalement une poignée (3) destinée à être saisie par un utilisateur, et la manipulation de la poignée actionne l'effecteur terminal, et
- le kit de parties selon la revendication 1,
la surface de réception (27) de la pince (5) recevant et pinçant au moins partiellement une partie de la poignée (3) ou une partie de l'instrument chirurgical distalement adjacent à la poignée (3), mettant ainsi en prise de manière libérable la pince (5) et l'instrument chirurgical manuel, et le dispositif (1) et ledit élément de support (20) étant interconnectés par les éléments de connexion s'emboîtant mutuellement (10, 9) respectivement fournis sur celle-ci.

3. Système ou kit de pièces selon les revendications 1 ou 2, dans lequel l'élément de logement (19) s'étend circonférentiellement avec au moins une partie de la surface de réception (27) de la pince (5).

4. Système ou kit de pièces selon la revendication 3, dans lequel l'élément de support (20) est amovible le long d'au moins une partie de la circonférence de l'élément de logement respectif (19).

5. Système ou kit de pièces selon l'une quelconque des revendications 1 à 4, dans lequel l'élément de support (20) est amovible entre deux positions discrètes ou plus sur l'élément de logement (19), ou est continuellement amovible le long d'un espace circonférentiel donné de l'élément de logement (19).

6. Système ou kit de pièces selon l'une quelconque des revendications 1 à 5, dans lequel l'élément de support (20) est engagé par coulissement avec l'élément de logement (19).

7. Système ou kit de pièces selon l'une quelconque des revendications 1 à 6, dans lequel l'élément de support (20) définit un corps faisant généralement saillie radialement vers l'extérieur à partir de l'élément de logement (19), ledit corps définissant une extrémité radialement proximale (34), une extrémité radialement distale (23) et une partie tige interposée (36) connectant lesdites extrémités.

8. Système ou kit de pièces selon la revendication 7, dans lequel l'élément de connexion (9) est fourni au niveau de l'extrémité radialement distale (23) de l'élément de support (20) ou adjacent à celle-ci.

9. Système ou kit de pièces selon la revendication 8, dans lequel l'élément de connexion (9) est amovible relativement à l'élément de support (20) sur lequel il est fourni.

10. Système ou kit de pièces selon l'une quelconque des revendications 1 à 9, dans lequel les éléments de connexion (9, 10) sont configurés pour une interconnexion amovible, de préférence coulissante, entre l'élément de support (20) et le dispositif (1).

11. Système ou kit de pièces selon l'une quelconque des revendications 1 à 10, dans lequel le dispositif (1) comprend au moins une paire, par exemple 2 paires, 3 paires ou plus, d'éléments d'entrée (2) configurés pour produire des signaux identiques, chaque membre de ladite paire étant positionné de manière substantiellement symétrique sur les côtés latéraux opposés (4) du dispositif (1) ; de préférence chaque paire étant configurée pour produire des signaux différents de ceux de toute autre paire.

12. Système ou kit de pièces selon l'une quelconque des revendications 1 à 10, dans lequel le dispositif (1) comprend au moins une paire d'éléments d'entrée (2) configurés pour produire des signaux identiques sur la même surface latérale (4) du dispositif (1).

13. Système ou kit de pièces selon l'une quelconque des revendications 1 à 12, dans lequel ladite commande sans fil est effectuée par un système de transmetteur-récepteur, de préférence où le dispositif comprend le transmetteur et l'appareil, ou un second appareil configuré pour commander ledit appareil, comprend le récepteur.

14. Système ou kit de pièces selon l'une quelconque des revendications 1 à 13, dans lequel le dispositif est configuré pour commander le mouvement spatial de l'appareil distant ou d'une partie de celui-ci.

15. Système ou kit de pièces selon l'une quelconque des revendications 1 à 14, dans lequel l'appareil distant est choisi dans le groupe comprenant un système de positionnement pour un instrument chirurgical, tel qu'un système de positionnement pour des systèmes de visualisation laparoscopiques et/ou endoscopiques, ou un système de positionnement pour des plateformes portant des charges.

16. Système ou kit de pièces selon l'une quelconque des revendications 1 à 14, dans lequel le dispositif est configuré pour commander une commande informatique effectuée par l'appareil distant.

17. Système ou kit de pièces selon l'une quelconque des revendications 1 à 16, dans lequel la manipulation du ou des éléments d'entrée entraîne la production par le dispositif de signaux adaptés pour commander l'appareil distant.

18. Système ou kit de pièces selon l'une quelconque des revendications 1 à 17, dans lequel la manipulation comprend la réalisation d'un mouvement spatial des et/ou l'application de pression sur le ou les éléments d'entrée ou des parties de ceux-ci.

19. Système ou kit de pièces selon l'une quelconque des revendications 1 à 18, dans lequel le ou les éléments d'entrée sont positionnés de manière à ce que lorsque le dispositif est monté sur l'instrument chirurgical manuel, l'élément d'entrée puisse être manipulé par au moins un doigt d'une main tenant l'instrument chirurgical manuel.

20. Système ou kit de pièces selon l'une quelconque des revendications 1 à 19, dans lequel l'élément d'entrée est choisi dans le groupe comprenant une manette, un commutateur à bouton pressoir, un pavé tactile ou une boule de commande.

21. Système ou kit de pièces selon la revendication 20, dans lequel la manette est configurée pour actionner un mouvement spatial de l'appareil distant ou d'une partie de celui-ci le long de 1 à 3 axes orthogonaux.

22. Système ou kit de pièces selon la revendication 20, dans lequel le dispositif comprend au moins une manette configurée pour actionner le mouvement spatial de l'appareil distant ou d'une partie de celui-ci le long de 2 axes orthogonaux et au moins une manette configurée pour actionner le mouvement spatial de l'appareil distant ou d'une partie de celui-ci le long du troisième axe orthogonal.

23. Système ou kit de pièces selon l'une quelconque des revendications 1 à 22, dans lequel l'instrument chirurgical est un instrument chirurgical laparoscopique.

24. Système ou kit de pièces selon l'une quelconque des revendications 1 à 23, qui est adapté pour être stérilisé ou être jeté.
